# EUROPÄISCHE PATENTSCHRIFT

(11) **EP 2 295 086 B1**
(45) Veröffentlichungstag und Bekanntmachung des Hinweises auf die Patenterteilung: **18.01.2012**
(21) Anmeldenummer: 10150773.9
(22) Anmeldetag: 03.11.2008
(51) Int. Cl.: A61L 2/00, C07K 14/435, C12N 9/94

(54) **Verfahren zur Verringerung der viralen und mikrobiellen Belastung von Pankreatin**
Method of reducing the viral and microbial load of pancreatin
Procédé destiné à réduire la charge virale et microbienne de pancréatine

(43) Veröffentlichungstag der Anmeldung: 16.03.2011
(62) Teilanmeldung aus: 08019210.7
(73) Patentinhaber: Nordmark Arzneimittel GmbH & Co.KG, 25436 Uetersen (DE)
(72) Erfinder: Kurfürst, Manfred, Dr., 25436 Moorrege (DE); Rämsch, Christian, Dr., 25436 Groß Nordende (DE); Schräder, Thomas, Dr., 25436 Uetersen (DE); Moest, Thomas, Dr., 25436 Moorrege (DE)
(74) Vertreter: Richter, Werdermann, Gerbaulet & Hofmann

(56) Entgegenhaltungen:
- EP-A- 0 583 726
- WO-A-2007/014896
- CA-A- 394 981
- DE-A1- 2 106 706
- US-A- 3 844 891

## Beschreibung

### Anwendungsgebiet

Die Erfindung betrifft ein Verfahren zur Herstellung von Pakreatin mit verringerter viraler und mikrobieller Belastung.

### Stand der Technik

Pankreatin wird als Extrakt aus tierischen Pankreas-Drüsen gewonnen. Derartige Extrakte, die aus biologischem Ausgangsmaterial gewonnen werden, können eine hohe Virenbelastung aufweisen. Viren sind Nukleinsäuren, die von einer Proteinhülle umgeben sind. Bei behüllten Viren kommt noch eine äußere Lipidhülle hinzu. Da sich Viren nicht eigenständig replizieren können, sind sie auf Wirte angewiesen. Dem entsprechend kommen sie in praktisch allen Lebewesen der Erde vor. Die wenigsten der bekannten Viren sind für den Menschen pathogen, da sie eine hohe Wirtsspezifität besitzen. Um eine Gefährdung der Konsumenten von vorn herein auszuschließen, sollten Extrakte, die für den menschlichen Verzehr bestimmt sind oder die als Wirkstoff in Arzneimitteln eingesetzt werden, grundsätzlich eine möglichst geringe bzw. keine Virenbelastung aufweisen. Nicht immer führt das eigentliche Produktionsverfahren zu einer signifikanten Inaktivierung oder Entfernung der vorhandenen Viren, so dass, insbesondere bei der Herstellung pharmazeutischer Wirkstoffe, zusätzliche Abreicherungs- oder Inaktivierungsschritte in das Verfahren integriert werden müssen.

Für die Abreicherung oder Inaktivierung von Viren und Mikroorganismen sind zahlreiche Methoden beschrieben [K.H. Wallhäuser, Praxis der Sterilisation Desinfektion Konservierung, Thieme Verlag, Stuttgart 1995]. Neben der mechanischen Entfernung durch z. B. Chromatographie oder Filtration können diese Kontaminationen durch Zusatz chemischer Verbindungen selektiv inaktiviert werden. Letzteres Verfahren ist aber in sofern problematisch, als diese Verbindungen wieder vollständig entfernt werden müssen, damit sie im Endprodukt keine toxischen Effekte hervorrufen. Physikalische Methoden, wie z. B. Hitzebehandlung oder Bestrahlung sind ebenfalls gängige Verfahren zur Inaktivierung von Viren oder Mikroorganismen.

Eine besondere Herausforderung ist die Inaktivierung oder Entfernung von Viren aus komplexen biologischen Extrakten, deren Wirksubstanz Enzymgemische sind, ohne dabei die enzymatische Aktivität der Proteine zu zerstören oder zu verändern.

Von besonderem wirtschaftlichem Interesse ist der pharmazeutische Wirkstoff Pankreatin, der als Extrakt aus der Schweinepankreas gewonnen wird und in getrockneter Form als orales Therapeutikum Anwendung findet, wie in DE 3248588 A1 beschrieben.

Die Wirksubstanzen im Pankreatin sind u. a. verschiedene polymerabbauende Enzyme, wie Lipasen, Amylasen und Proteasen. Eine Voraussetzung für die Wirksamkeit des Therapeutikums ist, dass alle Enzyme in einem bestimmten Verhältnis und in aktiver Form im Wirkstoff vorhanden sind.

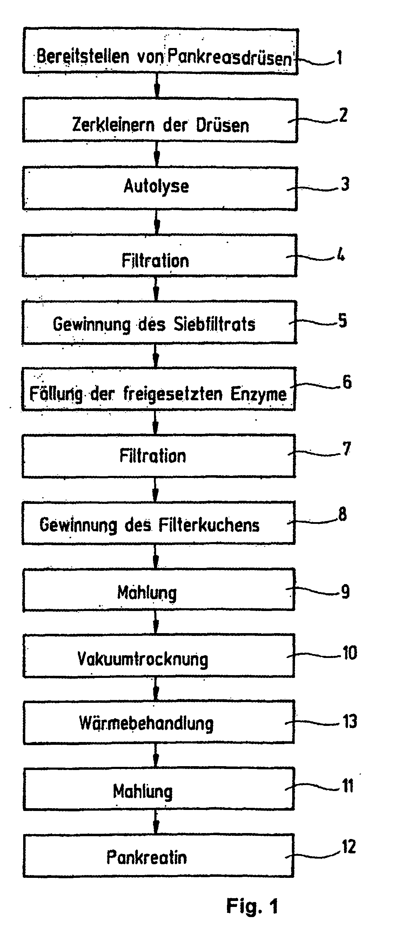

Untersuchungen zur Virusbelastung von Pankreatin haben gezeigt, dass porcines Parvovirus (PPV) als einziges Virus im Pankreatin als infektiöses Partikel nachweisbar ist. Die zoonotischen Viren EMCV, PEV9 und HEV sowie Rotavirus und Reovirus konnten weder als infektiöse Partikel noch auf Genomebene nachgewiesen werden. Grundsätzlich sollten pharmazeutische Wirkstoffe keine infektiösen Viren enthalten. Obwohl PPV nach derzeitigem Kenntnisstand für den Menschen nicht pathogen ist, sollte das Ziel deshalb ein PPV-freies Pankreatin sein. PPV ist ein häufig verwendetes Modelvirus, da es sich durch eine sehr hohe Resistenz gegenüber verschiedensten Inaktivierungsverfahren auszeichnet. Da der in Fig. 1 beschrieben Produktionsprozess nicht in der Lage ist, die vorhandene PPV-Belastung vollständig zu entfernen, müssen zusätzliche virenreduzierende Schritte implementiert werden.

Klassische vireninaktivierende Methoden wie z. B. trockene oder feuchte Hitze oder virenabreichernde Methoden wie z. B. Filtration oder Chromatographie lassen sich bei Extrakten aus biologischem Ausgangsmaterial und insbesondere bei Organextrakten ohne Veränderung der Zusammensetzung und/oder hohe Produktverluste in den meisten Fällen nicht anwenden.

Aus US 3,956,483 A ist ein Verfahren zur Herstellung einer Pankreatin-haltigen Zusammensetzung in trockener Pulverform bekannt, bei dem zerkleinerter Pankreas mit einer wässerigen Lösung versetzt wird, die Calciumsulfat enthält. Diesem Gemisch wird dann ein Enzymaktivator zugesetzt. Nach einem vorgegebenen Zeitraum für die Enzymaktivierung wird das Gemisch schließlich dehydriert, wodurch pathogene Bakterien inaktiviert werden sollen. Diese Inaktivierung soll bei Temperaturen über 160 °F (73 °C), vorzugsweise bei 180 °F (82 °C) durchgeführt werden. In sämtlichen Beispielen wird eine Inaktivierungs-Temperatur von 82°C verwendet.

US 2007/0148151 A1 offenbart ein Verfahren zur Herstellung von Pankreatin. Dieses Verfahren sieht zur Verringerung der Viren- und Bakterienbelastung eine Erwärmung von Pankreatin in disperser Form vor, wozu das Pankreatin weniger als 9 Gew.-% eines oder mehrerer Lösungsmittel enthalten soll. Die Beispiele zeigen Verfahren, bei denen der Anteil des Lösungsmittels mindestens 1 Gew.-% betrug. Das disperse Pankreatin wird dann auf eine Temperatur von mindestens 85 °C für einen Zeitraum von weniger als 48 h erwärmt.

Eine Behandlung bei 85 °C oder noch höheren Temperaturen ist gemäß US 2007/0148151 A1 erforderlich, um die amtlichen Vorschriften zur Virusbelastung von biologischen Erzeugnissen erfüllen zu können. Bei einer Temperatur von 80° C könnten die Vorschriften nicht erfüllt werden.

In US 2007/0148151 A1 ist ferner angegeben, dass eine Wärmebehandlung bei 60°C für 70 Stunden Pankreatin schädigen kann. Eine Wärmebehandlung könne eine wesentliche Menge der enzymatischen Aktivität des Pankreatins zerstören.

Die WO 2007/014896 A betrifft Prozesse zur Herstellung von sterilisiertem Pakreatinpulver. Der Prozess zur Herstellung von Pankreatin umfasst die Erwärmung einer dispergierten Form von Pankreatin als Ausgangsprodukt, das ein oder mehreren Lösungsmittel enthält, auf eine Temperatur von mindestens 85°C, wobei ein Gesamtlösungsmittelgehalt in der dispergierten Form von Pankreatin von weniger als 9 % Massenanteil an jedem Punkt wähend des Erwärmungsschrittes erreicht werden soll. Das Vorerwärmen der dispergierten Form des Pankreatins, das ein oder mehrere Lösungsmittel enthält, erfolgt dabei auf eine Temperatur von mindestens 85°C. Die Erwärmung der dispergierten Form von Pankreatin auf eine Temperatur von mindestens 85°C für eine Zeit von bis zu 48 Stunden wird dann fortgesetzt, so dass ein Gesamtlösungsmittelgehalt in der dispergierten Form von Pankreatin von weniger als 9 % Massenanteil an jedem Punkt während der Erwärmung der dispergierten Form von Pankreatin erreicht wird, wobei diese Vorgabe sich nicht auf den Schritt der Vorerwärmung erstreckt.

Nach diesem Verfahren soll der Gesamtlösungsmittelgehalt während der Dauer der beiden Erwärmungsschritte (Vorerwärmung / Haupterwärmung) der dispergierten Form von Pankreatin von weniger als 9 % Massenanteil gehalten werden.

Die US 3,844,891 A offenbart ein keimarmes fließfähiges Pankreatinpräparat, das zu ungereinigtem PAnkreatin eine unveränderte Enzymaktivität aufweist, und ein Verfahren zu dessen Herstellung. Dieses keimarme und gut fließfähige Pankreatinpräparat mit einer unveränderten Enzymaktivität wird dadurch erhalten, dass ein kontaminiertes Pakreatinpräparat mit einer wässrigen Lösung oder mit einer stabilisierten Emulsion eines niederaliphatischen Ketons, vorzugsweise mit 3 bis 6 Kohlenstoffatomen, in erster Linie Butan-2-on, oder eines halogenierten Kohlenwassersoffs mit einem Siedepunkt zwischen 15°C und 100°C, insbesondere 30°C bis 60°C, in erster Linie Methylenchlorid oder Chloroform in Anwesenheit eines Verdickungsmittels zu einer plastischen Masse verarbeitet wird und diese in zerkleinertem Zustand bei einer Temperatur unterhalt 40°C trocknet.

Mit diesem Verfahren soll ein keimarmes Granulat mit ausgezeichneten Fließeigenschaften erhalten werden, das leicht zu festen Darreichungsformen verarbeitet werden kann.

Die DE-A-2106706 beschreibt ein Verfahren zur Herstellung von Pankreatin als trockenes Pulver mit hoher proteolytischer, amylolytischer und lipolytischer Aktivität. Dieses Verfahren besteht darin, dass zu zerkleinertem Schweine- oder Rinderpankreas unter Mischen eine wässrige Lösung von Calciumsulfat und/oder Calciumacetat in einer Menge von 5,5 Gew.-% Wasser und 0,02 bis 1,4 Gew.-% Salz bezogen auf das zerkleinerte Pankreas, zugegeben wird, zu diesem Gemisch ungefährt 10 %, bezogen auf das Gewicht des zerkleinerten Pankreas, zerkleinertes Schweine-Duodenum zugegeben wird, das Gemisch bis zu 7 Tagen auf einer Temperatur von ca. 3,3°C bis 4,4°C gehalten wird, mit Aceton gewaschen und das gewaschene Gemisch auf einen Temperaturbereich von 49°C bis 82°C erhitzt wird. Das getrocknete Produkt wird anschließend vermahlen. Angaben zu einem Restfeuchtegehalt des nach diesem Verfahren hergestellten Produkts werden nicht gemacht.

Aus dem Stand der Technik ist ferner bekannt, dass die Ergebnisse einer Virenreduktion mittels Einwirkung von Hitze erheblich von der Restfeuchte der Proben abhängen. Je trockener das Material, das der Erhitzung unterzogen wird, desto höher sei die Resistenz der Viren gegenüber hohen Temperaturen.

Daraus folge, dass die Temperatur der Erhitzung umso geringer gewählt werden kann, je höher die Restfeuchte des Materials ist.

### Aufgabe, Lösung, Vorteil

Aufgabe der Erfindung ist es, ein Verfahren zur Herstellung von Pankreatin mit verringerter viralen und mikrobiellen Belastung bei Aufrechterhaltung der Enzymaktivität und mit einem geringen Restfeuchtegehalt zur Verfügung zu stellen.

Diese Aufgabe wird durch die Merkmale des Anspruchs 1 gelöst. Zweckmäßige Ausgestaltungen der Erfindung ergeben sich aus den Merkmalen der Unteransprüche.

Danach besteht gemäß Anspruch 1 die Erfindung in einem Verfahren zur Herstellung von Pankreatin in gemahlener Form als Pulver mit einer Restfeuchte von 0,1 bis 0,3 Gew.-%, das folgende Schritte umfasst:
a) Zerkleinerung von von Hausschweinen stammenden Pankreasdrüsen und Unterziehung einer Autolyse vermittels eines Lösungsmittels oder mehrerer Lösungsmittel, wie wässriges Isopropanol, Wasser, Aceton oder Gemischen davon,
   a1) Filtration des nach Schritt a) erhaltenen Zwischenprodukts, zur Gewinnung eines Siebfiltrates,
   a2) Ausfällung der in dem durch Filtration nach Schritt a1) erhaltenen Siebfiltrates enthaltenen Enzyme,
   a3) Filtrierung des Gemisches aus Schritt a3) und Gewinnung eines Filterkuchens,
   a4) Vermahlung und anschließende Vakuumtrocknung des Filterkuchens bis zu einer Restfeuchte von 0,1 bis 0,3 Gew.-%,
   a5) Wärmebehandlung des Filterkuchens mit trockener Hitze bei einer Temperatur von 70°C bis 80°C, bevorzugterweise bei 80°C, und in 1h bis 48h oder 8h bis 48h oder 24h bis 48h oder 32h bis 48h,
   a6) Vermahlung des wärmebehandelten Filterkuchens zum fertigen PAnkreatin mit einer Restfeuchte von 0,1 bis 0,3 Gew.-%.

Die Restfeuchte des in Schritt (a6) erhaltenen Pankreatins beträgt 0,1 bis 0,3 Gew.-%. Die Wärmebehandlung in Schritt (a5) wird bevorzugterweise bei 80° C durchgeführt.

Das unbehandelte Pankreatin ist dabei aus der Pankreas des Schweins gewonnen.

Nach dem erfindungsgemäßen Herstellungsverfahren wird ein Pankreatin erhalten, bei dem die biologische Aktivität des wärmebehandelten Pankreatins zumindest 50 % der biologischen Aktivität des unbehandelten Pankreatins entspricht, die virale Infektiosität des wärmebehandelten Pankreatins um einen Faktor von größer als 1 log₁₀ im Vergleich zur viralen Infektiosität des unbehandelten Pankreatins verringert worden ist und die Keimbelastung des wärmebehandelten Pankreatins kleiner als 500 KBE/g ist.

### Kurzbeschreibung der Zeichnung

Die Erfindung wird nachfolgend unter Bezugnahme auf die Zeichnungen näher erläutert. Dabei zeigen:
- Fig. 1: ein Ablaufschema eines erfindungsgemäßen Verfahrens zur Herstellung von Pankreatin aus Schweine-Pankreasdrüsen;
- Fig. 2: ein Diagramm, das die Lipaseaktivität von behandelten Pankreatin- Proben nach unterschiedlichen Behandlungszeiten zeigt;
- Fig. 3: ein Diagramm, das den Virustiter von behandelten Pankreatin-Proben nach unterschiedlichen Behandlungszeiten zeigt;
- Fig. 4: ein Diagramm, das den Lipaseaktivitätsverlust bei unterschiedlichen Restfeuchten der behandelten Pankreatin-Proben zeigt; und
- Fig. 5: ein Diagramm, das den Virustiter von behandelten Pankreatin-Proben nach unterschiedlichen Behandlungszeiten zeigt.

### Detaillierte Beschreibung der Erfindung und bester Weg zur Ausführung der Erfindung

Erfindungsgemäß ist die Wärmebehandlung von festem Pankreatin vorgesehen, das eine Restfeuchte von 0,1 bis 0,3 Gew.-% aufweist. Es wurde aufgefunden, dass im Gegensatz zu den Annahmen, die im Stand der Technik getroffen worden sind, auch bei einer derart geringen Restfeuchte eine wirksame Reduktion der viralen und mikrobiellen Belastung erreicht werden kann.

Überdies wurde festgestellt, dass die Behandlung von Pankreatin mit der angegebenen Restfeuchte bei einer Temperatur erfolgen kann, die bevorzugterweise 80° C oder weniger beträgt. Eine geringere Behandlungstemperatur ist neben dem ökonomischen Vorteil insbesondere mit einer besseren Bewahrung der ursprünglichen enzymatischen Aktivität der Pankreatin-Enzyme verbunden. Durch die erfindungsgemäß vorgesehene Verringerung des Restfeuchtegehaltes konnte der Verlust der Enzymaktivität (< 20 %) gegenüber dem Stand der Technik erheblich reduziert werden.

Unter dem Begriff "Restfeuchte" wird hier der Gehalt des Pankreatins an einem oder mehreren Lösungsmitteln, beispielsweise Wasser, Aceton, Isopropanol oder Gemische davon, oder sonstiger Flüssigkeiten verstanden.

Eine Ausführungsform des erfindungsgemäßen Verfahrens zur Herstellung von Pankreatin mit verringerter viraler und bakterieller Belastung wird nachstehend unter Bezugnahme auf Fig. 1 beschrieben. Die von Hausschweinen stammenden Pankreasdrüsen 1 werden zunächst zerkleinert 2 und einer Autolyse 3 unterzogen. Durch Filtration 4 des so erhaltenen Zwischenproduktes wird das Siebfiltrat gewonnen 5. Anschließend werden die Enzyme, die sich in dem Siebfiltrat befinden, ausgefällt 6, das Gemisch filtriert 7 und der Filterkuchen gewonnen 8. Der Filterkuchen wird schließlich gemahlen 9 und vakuumgetrocknet 10, bis er eine Restfeuchte, die 0,1 bis 0,3 Gew.-% beträgt, aufweist. Anschließend wird der Filterkuchen einer Wärmebehandlung bei 80° C oder weniger unterzogen 13. Der wärmebehandelte Filterkuchen wird dann erneut gemahlen, wodurch das fertige Pankreatin erhalten wird 12.

Der gemäß dem vorstehenden Verfahren gewonnene Filterkuchen (Bezugszeichen 8 in Fig. 1) enthält noch einen Teil des Lösungsmittels, das zur Autolyse der zerkleinerten Pankreas-Drüsen verwendet wurde (Bezugszeichen 3 in Fig. 1). Dieses Lösungsmittel ist in der Regel wässeriges Isopropanol. Trotz der Filtration enthält der gewonnene Filterkuchen verfahrensbedingt noch einen Teil des Lösungsmittels, der durch die Vakuumtrocknung weiter verringert und dabei auf einen Anteil von 0,1 bis 0,3 Gew.-%, bezogen auf das durch die Vakuumtrocknung erhaltene Pankreatin, eingestellt wird.

Der Begriff "Pankreatin" umfasst in der vorliegenden Erfindung hier den mit Bezugszeichen 8 bezeichneten Filterkuchen, bei dem es sich um Roh-Pankreatin handelt, dass gemäß der Erfindung weiteren Behandlungsschritten (Bezugszeichen 9 bis 11 und 13 in Fig. 1) unterzogen wird. Dieser Filterkuchen wird auch als "unbehandeltes Pankreatin" bezeichnet. Der Begriff "Pankreatin" umfasst ferner das Pankreatin, dass dem Verfahrensschritt (a5), d.h. der Wärmebehandlung, unterzogen worden ist. Dieses Pankreatin wird hier auch als "behandeltes Pankreatin" bezeichnet.

Unter dem Ausdruck "in fester Form" wird Pankreatin verstanden, das nicht als Lösung, Emulsion oder Suspension vorliegt. Das Pankretin liegt in fester gemahlener Form, beispielsweise als Pulver vor.

Die Wärmebehandlung wird typischerweise in einem Ofen durchgeführt. Bevorzugt wird die Wärmebehandlung bei einer Temperatur von 70 bis 80° C, besonders bevorzugt bei 80° C durchgeführt. Die Temperatur sollte unterhalb von 84° C liegen. Die Wärmebehandlung sollte mit "trockener Hitze" erfolgen.

Die Wärmebehandlung wird vorzugsweise für einen Zeitraum von 48 h oder weniger durchgeführt. Bevorzugt wird die Wärmebehandlung 1 h bis 48 h, weiter bevorzugt 8 h bis 48h, stärker bevorzugt 24 bis 48 h und insbesondere 24 h, 32 h oder 48 h durchgeführt.

Die biologische Aktivität des in Schritt (b) erhaltenen Pankreatins sollte zumindest 80 %, bevorzugt zumindest 90 % der biologischen Aktivität des in Schritt (a5) bereitgestellten Pankreatins entsprechen. In bezug auf die Enzyme die im Pankreatin enthalten sind, entspricht der Begriff "biologische Aktivität" dem Begriff "enzymatische Aktivität".

Ferner sollte die Belastung des in Schritt (a5) erhaltenen Pankreatins mit toxischen Substanzen nicht höher als die Belastung des in Schritt (a) bereitgestellten Pankretins sein. Die Keimbelastung des in Schritt (a5) erhaltenen Pankreatins sollte kleiner als 500 KBE/g sein, bevorzugt nicht mehr als 100 KBE/g.

Eine bevorzugte Quelle, aus der unbehandeltes Pankreatin gewonnen werden kann, ist das Pankreas des Schweins.

Das erfindungsgemäße Verfahren ist auf alle Virusformen, insbesondere DNA-und RNA-Viren, behüllte und unbehüllte Viren, weiterhin auf Virionen und Prionen oder ähnliche biologische Systeme sowie Bakterien und Pilze anwendbar. Das Verfahren wird bevorzugt zur Verringerung der PPV-Belastung und/oder der Reoviren-Belastung von Pankreatin aus dem Schweine-Pankreas verwendet. Zusätzlich oder alternativ kann das Verfahren zur Verringung der Belastung an folgenden Viren eingesetzt werden: Pseudorabies-Virus (PRV), Bovines virales Diarrhoea-Virus (BVDV), Encephalomyocarditisvirus (EMCV).

Das erfindungsgemäße Verfahren erlaubt die Reduktion der Viren- und Mikroorganismen-Belastung von Pankreatin, ohne dass sich deren enzymatische Aktivität wesentlich verringert, die pharmakologisch beabsichtigten Eigenschaften verschlechtert oder toxische chemische Verbindungen erzeugt werden.

Die virale Infektiosität des wärmebehandelten Pankreatins sollte um einen Faktor von größer als 1 log₁₀, vorzugsweise größer 3 log₁₀, besonders bevorzugt größer 4 log₁₀, im Vergleich zur viralen Infektiosität vor der Wärmebehandlung verringert worden sein. Dies gilt insbesondere auch für die virale Infektiosität von porcinem Parvovirus (PPV) und /oder die virale Infektiosität von Reovirus. Beispielsweise sollte die PPV-Infektiosität im Pankreatin nach der Behandlung um einen Faktor von größer als 1 log₁₀, vorzugsweise größer 3 log₁₀, besonders bevorzugt größer 4 log₁₀, im Vergleich zu dessen Infektiosität vor der Behandlung verringert sein.

Die virale Infektiosität wird durch Endpunkttitration und anschließende Berechnung des Halbwertes der Gewebekultur-Infektionsdosis (TCID₅₀) gemäß der Spearman-Kärber-Formel berechnet (Bundesanzeiger, Nr. 84, 4. Mai 1994). Die so errechneten Virustiter werden als log₁₀ TCID₅₀ pro ml mit Konfidenzintervallen von 95 % angegeben.

Die Reduktion der viralen Belastung wird gemäß der EG-Richtlinie CMMP/BWP/268/95, Anhang II) als logarithmischer Reduktionsfaktor angegeben, der die Differenz der Virustiter zwischen einer Kontrollprobe und den wärmebehandelten Proben ist.

Die Restfeuchte wird nach bekannten Verfahren bestimmt.

Die Erfindung wird nachfolgend anhand eines Beispiels näher erläutert. Das Beispiel soll den Umfang der Erfindung in keiner Weise einschränken.

### Beispiele

Es wurden zahlreiche Experimente durchgeführt, um den Einfluss der Restfeuchte und der Temperatur, bei der die Wärmebehandlung durchgeführt wird, auf die biologische Aktivität und den Virustiter von festem Pankreatin zu untersuchen. Dabei wurde zunächst der Einfluss der Behandlungstemperatur auf die Pankreatinstabilität untersucht. Als Maß für die Pankreatinstabilität wurde die spezifische Lipaseaktivität bestimmt.

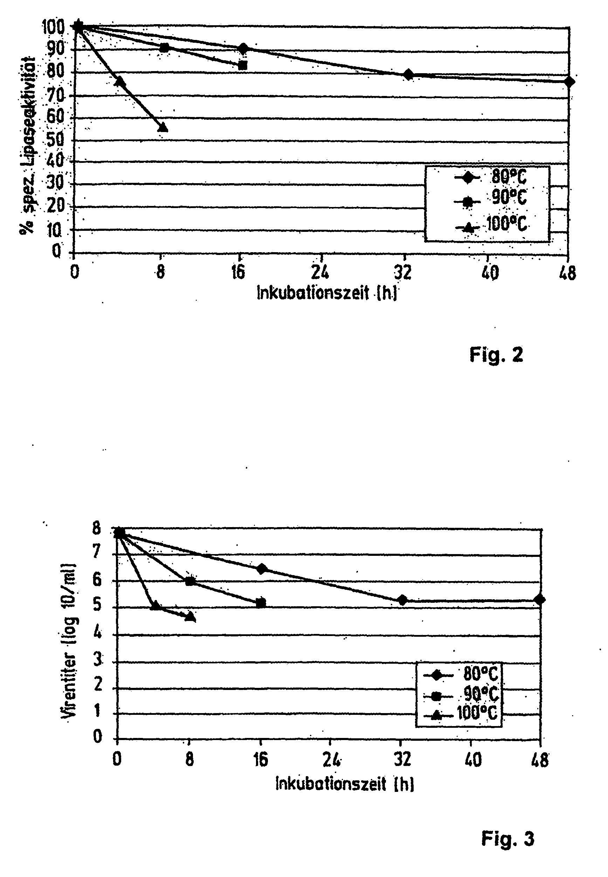

In Fig. 2 sind die Ergebnisse dieser Untersuchungen gezeigt. Die untersuchten Proben wiesen vor der Wärmebehandlung eine Restfeuchte von weniger als 3,0 Gew.-%, und zwar 1,7 Gew.-% auf. Diese Proben wurden bei Temperaturen von 80°C, 90°C und 100°C wärmebehandelt und die Lipaseaktivität nach 8h, 16h und 48 h bestimmt. Es ist in Fig. 2 zu erkennen, dass die Lipaseaktivität umso stärker und umso schneller abnimmt, je höher die Behandlungstemperatur ist.

In Fig. 3 ist der Einfluss der gewählten Behandlungstemperatur auf den Virustiter gezeigt. Wie zu erkennen ist, wird bei einer ausreichenden Behandlungszeit auch bei einer Behandlungstemperatur von 80° C eine hinreichende Abnahme des Virustiters erreicht.

In weiteren durchgeführten Versuchen wurde festgestellt, dass eine Inaktivierung von Viren, insbesondere von PPV und Reoviren, auch bei 80° C und einer sehr geringen Restfeuchte von 0,5 Gew-% oder weniger vor der Wärmebehandlung erreicht wird.

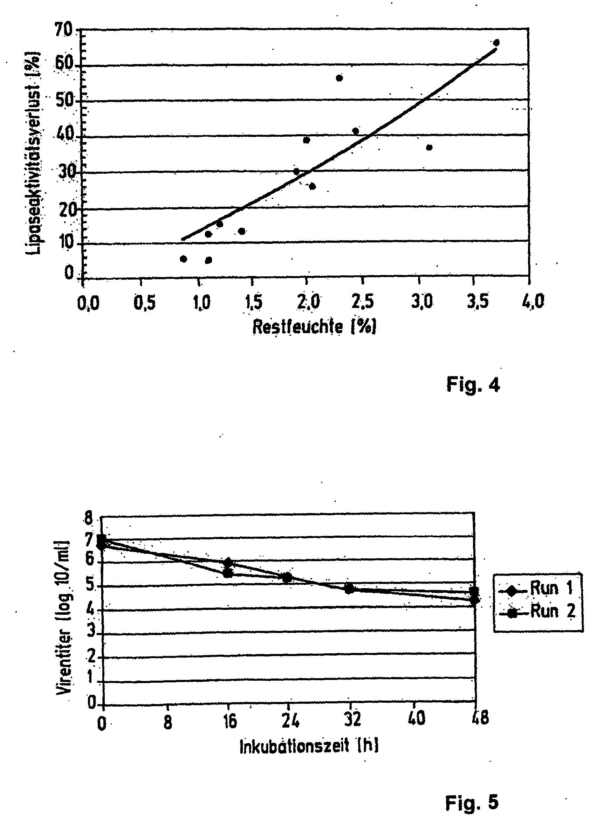

Fig. 4 zeigt ein Diagramm, das den Lipaseaktivitätsverlust von Pankreatin nach einer Wärmebehandlung von 24 h bei 80° C zeigt. Es ist zu erkennen, dass bei höheren Restfeuchten, die Wärmebehandlung mit einem höheren Aktivitätsverlust verbunden ist. Aus diesem Grund haben die Erfinder entschieden, die Wärmebehandlung von Pankreatin, das nur eine geringe Restfeuchte von 0,5 Gew-% oder weniger aufweist.

Die Ergebnisse der Untersuchung von Pankreatin-Proben, die mit PPV gespikt waren, sind in Fig. 5 gezeigt. Die Restfeuchte dieser Proben betrug 0,1 bis 0,3 Gew.-%. Der Einfluss der Wärmebehandlung bei 80° C auf PPV ist für zwei Versuchsreihen in Tabelle 1 dargestellt:

**Tabelle 1**

| Zeit [h] | Reihe Nr. 1¹ | Reihe Nr. 2² |
|---|---|---|
| | Reduktionsfaktoren | Reduktionsfaktoren |
| | [log_{10]} ± Standardabweichung | [log₁₀] ± Standardabweichung |
| 16 | 1,55 ± 0,41 | 0,88 ± 0,55 |
| 24 | 1,81 ± 0,40 | 1,49 ± 0,47 |
| 32 | 2,28 ± 0,38 | 2,01 ± 0,66 |
| 48 | 2,48 ± 0,71 | 2,55 ± 0,99 |

| | | |
|---|---|---|
| ¹ Restfeuchte vor der Behandlung: 0,1 bis 0,3 Gew.-% ² Restfeuchte vor der Behandlung: 0,2 bis 0,3 Gew.-% | | |

Ferner sind Pankreatin-Proben, die mit Reoviren (im Folgenden auch als "REO" bezeichnet) gespikt waren, untersucht worden. Die Restfeuchte dieser Proben betrug 0,2 bis 0,3 Gew.-%. Der Einfluss der Wärmebehandlung bei 80° C auf Reoviren ist für zwei Versuchsreihen in Tabelle 2 dargestellt:

**Tabelle 2**

| Zeit [h] | Reihe Nr. 1 | Reihe Nr. 2 |
|---|---|---|
| | Reduktionsfaktoren | Reduktionsfaktoren |
| | [log_{10]} ± Standardabweichung | [log₁₀] ± Standardabweichung |
| 32 | 1,77 ± 0,37 | 1,72 ± 0,31 |

Ferner wurden Vergleichsversuche unter denselben Verfahrensbedingungen mit Vergleichsproben durchgeführt, die sich lediglich in der Restfeuchte von den erfindungsgemäßen Proben unterschieden. Die Vergleichsproben wiesen eine Restfeuchte zwischen von 2,0 bis 3,0 Gew.-% auf (Tabelle 3).

**Tabelle 3**

| Virus | Reduktionsfaktor (log₁₀) | | |
|---|---|---|---|
| | Nach 24 h | Nach 32 h | Nach 48 h |
| REO-80°C Erhitzungsschritt | 3,22 +/- 0,34 | 1.14+/-0.34 | 3,13 +/- 0,31 |
| Restfeuchten < 3% | 4,13 +/- 0,89 | 1,05 +/- 0,31 | 3,22 +/- 0,34 |
| REO - 80° C Erhitzungsschritt | 1,59 +/- 0,37 | 1,77 +/- 0,30 | 0,87 +/- 0,35 |
| Restfeuchten < 0,5% | 1,42 +/- 0,29 | 1,72 +/- 0,37 | 0,91 +/- 0,28 |
| PPV - 80° C Erhit-zungsschritt | 1,31 +/- 0,42 | 2,53 +/- 0,47 | 2,44 +/- 0,48 |
| Restfeuchten < 3 % | 2,81 +/- 0,96 | 2,36 +/- 0,30 | 2,81 +/- 0,96 |
| PPV - 80° C Erhitzungsschritt | 1,81 +/- 0,40 | 2,28 +/- 0,38 | 2,24 +/- 0,71 |
| Restfeuchten < 0,5% | 2,00 +/- 0,47 | 2,01 +/- 0,66 | 2,55 +/- 0,99 |

| | | | |
|---|---|---|---|
| *geringere Analysenzahl (kein large volume plating) | | | |

Die Untersuchungen zur Virenabreicherung durch Erhitzen bei sehr niedrigen Restfeuchtegehalten des Pankreatins (< 0,5 %) ergab Reduktionsfaktoren, die im Bereich der Untersuchungen bei höheren Restfeuchtegehalten lagen. Damit konnte gezeigt werden, dass PPV auch bei geringen Restfeuchten effektiv inaktiviert wird und gleichzeitig die Enzymaktivität weniger stark verringert wird.

Die Inaktivierung von Reovirus ist dagegen stärker abhängig von der Restfeuchte des getrockneten Pankreatins. Inaktivierungen bei Restfeuchten von 2,5% (<3%) ergaben Reduktionsfaktoren > 3 (24 und 48 Stunden), während für die Hitzebehandlung bei Restfeuchten von 0,2 bis 0,3 % (< 0,5 %) Reduktionsfaktoren im Bereich von 1,75 log Stufen (32 Stunden) ermittelt wurden. Dennoch bleibt der Einfluss trockener Hitze auch bei niedriger Restfeuchte auf die Abreicherung von Reovirus signifikant.

Die Ergebnisse zeigen, dass das erfindungsgemäße Verfahren eine signifikante Virusabreicherung gewährleistet.

### Probenherstellung und verwendete Testverfahren

### Wärmebehandlung von PPV-gespikten Proben mit einer Restfeuchte von 0,5 Gew.-% oder weniger

Zunächst wurden unbehandelte Pankreatin-Proben mit Viren gespikt. Anschließend wurden die gespikten Pankreatin-Proben lyophilisiert, um so die notwendige Restfeuchte einzustellen. Die dann erhaltenen Proben wurden schließlich einer Wärmebehandlung unterzogen.

### Lyophilisierungsverfahren

1,5 g Ausgangsmaterial Pankreatin (ECSM Ch.: 0677 005-P, Nordmark Arzneimittel GmbH & Co. KG) wurden mit 3,2 ml PBS resuspendiert und verwirbelt. Die Phiolen wurden dann mit 0,3 ml Virus gespikt. Phiolen zur Temperaturkontrolle und zur Bestimmung der Restfeuchte wurden mit 0,3 ml Virusverdünnungsmittel versehen. Der Inhalt aller Phiolen wurde dann mit einem Magnetrührer vermischt. Die Phiolen wurden in einen Gefriertrockner, eingestellt auf -30 °C, gegeben, um eine entsprechende Temperatur von -25 °C über Nacht zu erhalten.

Die Lyophilisierung wurde unter Vakuum bei 20 °C für 16 Stunden durchgeführt, worauf eine Erhöhung der Temperatur auf 50 °C für 24 Stunden folgte. Die Phiolen wurden unter Vakuum verschlossen. Eine Probe wurde direkt nach Ende der Lyophilisierung titriert und dazu in 5 ml PBS gelöst. Eine 1 :2000-Verdünnung wurde an allen Proben (mit Virusverdünnungsmittel) durchgeführt, um die vollständige Auflösung zu erreichen.

### Bestimmung der Restfeuchte

Die Restfeuchten der Proben vor der Wärmebehandlung wurden nach bekannten Verfahren bestimmt.

### Wärmebehandlung

Die Wärmebehandlung wurde in einem Ofen, eingestellt bei 80° C ± 1°C, durchgeführt. Sobald die Ofentemperatur 79 °C erreichte, wurden die Proben im Ofen platziert und dort für die vorgegebenen Zeiträume belassen. Die Temperatur wurde während des Verfahrens überwacht.

Die Proben wurden zur unmittelbaren Titrierung an den bezeichneten Zeitpunkten entnommen. Die Proben wurden direkt titriert und dazu in 5 ml PBS gelöst. Eine 1 : 2000-Verdünnung wurde bei allen Proben (mit Virusverdünnungsmittel) durchgeführt, um die vollständige Auflösung zu erreichen. Die Proben wurden auf Eis vor der Titrierung abgekühlt.

### Assayverfahren (Indirekter TCID₅₀-Assay)

PK-13-Zellen wurden in 96-Loch-Kulturplatten beimpft. Außerdem wurden zwei weitere 96-Lochplatten hinsichtlich negativer und positiver Kontrollen beimpft. Acht Löcher wurden mit jeder Verdünnung der Probe (25 µl/Loch) inokuliert. Nach der Inkubation für 70 Minuten ± 10 Minuten bei 36 °C ± 2 °C wurden den Platten 175 ml Zellwachstumsmedium (der Impfstoff wurde nicht entfernt) zugeführt. Der Assay wurde gemäß SOP EPBT0062 durchgeführt, und die Platten wurden hinsichtlich des TCID₅₀-Endpunktes als cytophatogener Effekt (CPE), entwickelt in der positiven Kontrolle, bewertet. Der TCID₅₀-Endpunkt wurde gemäß dem Spearman-Kärber-Verfahren berechnet, wie in SOP EPBT0062 beschrieben.

Die Ergebnisse sind in den Tabellen 1, 2 und 3 sowie Fig. 5 gezeigt.

## Patentansprüche

1. Verfahren zur Herstellung von Pankreatin in fester gemahlener Form als Pulver mit verringerter viraler und mikrobieller Belastung umfassend folgende Schritte:
a) Zerkleinerung von von Hauschweinen stammenden Pankreasdrüsen und Unterziehung einer Autolyse vermittels eines oder mehrerer Lösungsmittel, wie wässriges Isopropanol, Wasser, Aceton oder Gemischen davon,
a1) Filtration des nach Schritt a) erhaltenen Zwischenproduktes, zur Gewinnung eines Siebfiltrates,
a2) Ausfällung der in dem durch Filtration nach Schritt a1) erhaltenen Siebfiltrate enthaltenen Enzyme,
a3) Filtrierung des Gemisches aus Schritt a2) und Gewinnung eines Filterkuchens,
a4) Vermahlung und anschließende Vakuumtrocknung des Filterkuchens bis zu einer Restfeuchte von 0,1 bis 0,3 Gew.-%
a5) Wärmebehandlung des Filterkuchens mit trockener Hitze bei einer Temperatur von 70°C bis 80°C, bevorzugterweise bei 80°C und in 1h bis 48h oder 8h bis 48h oder 24h bis 48h oder 32h oder 48h,
a6) Vermahlung des wärmebehandelten Filterkuchens zum fertigen Pankreatin mit einer Restfeuchte von 0,1 bis 0,3 Gew.-%.

## Claims

1. A method for producing pancreatin in solid ground form as a powder having reduced viral and microbial load, said method comprising the following steps:
a) comminuting pancreas glands originating from domestic pigs and subjecting them to autolysis by means of one or more solvents, such as aqueous isopropanol, water, acetone or mixtures thereof,
a1) filtering the intermediate product obtained in accordance with step a) to obtain a screen filtrate,
a2) precipitating the enzyme obtained in the screen filtrate obtained by filtration in accordance with step a1),
a3) filtering the mixture from step a2) and obtaining a filter cake,
a4) grinding and then vacuum-drying the filter cake until a residual moisture of 0.1 to 0.3 % by weight is obtained,
a5) heat-treating the filter cake with dry heat at a temperature of 70 °C to 80 °C, most preferably at 80 °C and in 1 h to 48 h or 8 h to 48 h or 24 h to 48 h or 32 h or 48 h,
a6) grinding the heat-treated filter cake to form finished pancreatin having a residual moisture of 0.1 to 0.3 % by weight.

## Revendications

1. Procédé de production de pancréatine sous forme solide broyée avec charge virale et microbienne réduite, comportant les étapes suivantes :
a) fractionnement de glandes de pancréas émanant du cochon et soumission à une autolyse au moyen d'un ou de plusieurs solvants, comme de l'isopropanol aqueux, de l'eau, de l'acétone ou des mélanges de ces derniers,
a1) filtration du produit intermédiaire obtenu après l'étape a), pour récupérer un produit filtré au tamis,
a2) précipitation de l'enzyme contenu dans le produit filtré au tamis selon l'étape a1),
a3) filtrage du mélange issu de l'étape a2) et récupération d'un gâteau de filtre,
a4) broyage et séchage sous vide consécutif du gâteau de filtre jusqu'à une humidité résiduelle de 0,1 à 0,3 % en poids,
a5) traitement thermique du gâteau de filtre à la chaleur sèche, à une température comprise entre 70°C et 80°C, de préférence à 80°C et en de 1h à 48h ou de 8h à 48h ou de 24h à 48h ou de 32h à 48h,
a6) broyage du gâteau de filtre ayant subi un traitement thermique en pancréatine finie avec une humidité résiduelle de 0,1 à 0,3 % en poids.
